# EUROPEAN PATENT SPECIFICATION

(11) **EP 3 445 323 B1**
(45) Date of publication and mention of the grant of the patent: **09.12.2020**
(21) Application number: 17716190.8
(22) Date of filing: 05.04.2017
(51) Int. Cl.: A61K 8/34, A61K 8/37, A61Q 15/00, A61K 8/86, A61K 8/92

(54) **ANTIPERSPIRANT COMPOSITION**
SCHWEISSHEMMENDE ZUSAMMENSETZUNG
COMPOSITION ANTI-TRANSPIRANTE

(30) Priority: 19.04.2016 EP 16165958
(43) Date of publication of application: 27.02.2019
(73) Proprietor: Unilever PLC, London, Greater London EC4Y 0DY (GB); Unilever N.V., 3013 AL Rotterdam (NL)
(72) Inventor: FLANAGAN, Mark, Wirral Merseyside CH63 3JW (GB); JONES, Kimberley, Ann, Wirral Merseyside CH63 3JW (GB); MALHI, Laura, Louise, Rugby Warwickshire CV23 8UN (GB); RYMER, Sarah, Jane, Wirral Merseyside CH63 3JW (GB); SERRIDGE, David, Wirral Merseyside CH62 2FD (GB)
(74) Representative: Chisem, Janet
(86) International application number: PCT/EP2017/058163
(87) International publication number: WO 2017/182277

(56) References cited:
- EP-A1- 1 250 915
- WO-A1-2005/087188
- US-A- 3 755 590
- US-A1- 2003 138 389
- US-A1- 2004 247 547
- US-A1- 2006 182 773
- US-A1- 2014 364 394

## Description

### FIELD OF THE INVENTION

The invention relates to an antiperspirant composition, more particularly it relates to an aqueous antiperspirant composition.

### BACKGROUND OF THE INVENTION

Antiperspirant products are designed to control sweating. They include an antiperspirant active which is usually dissolved or dispersed in a carrier material. This formulation is in turn dispensed from a pack to the consumer's body, usually to the underarm area.

### SUMMARY OF THE INVENTION

There is a problem that for some aqueous antiperspirant compositions, they do not exhibit the required thermal stability at high temperatures. This improvement is particularly striking at temperatures around 50°C and above. Such a problem with thermal stability means that it is difficult to effectively formulate a product suitable for global application. The thermal instability of the product at higher temperatures also leads to a product that can be less effective at its primary role of sweat control.

However, the product should also have other characteristics that are also acceptable. One such characteristic is product rheology, for example, the viscosity. It is desired that the aqueous antiperspirant compositions of the invention have acceptable rheology so that they can be delivered to the consumer via a roll-on antiperspirant produce package.

It is an object of the invention to improve the thermal stability of aqueous antiperspirant compositions, whilst also allowing acceptable product viscosity so as to allow the product to be delivered to the consumer via a roll-on antiperspirant produce package.

We have now found that the thermal stability of an aqueous antiperspirant composition can be improved by utilising a certain combination of materials, namely a glyceryl mono- or di- ester, a fatty alcohol and glycerol in specified amounts and weight ratios. This combination of ingredients, especially the ratio of glyceryl mono- or di- ester to glycerol provides a product that has increased thermal stability but also displays excellent rheology in relation to product viscosity.

Document US2006/182773 provides non-tacky microemulsions, based on the same ingredients, but without glycerol.

The combination of ingredients allows for improved thermal stability, such as stability for 7 days at 50°C or even 55°C without phase separation. It also provides excellent product viscosity

There is provided a roll-on product comprising : a roll-on deodorant package comprising a roll-ball for dispensing an aqueous antiperspirant composition, the aqueous antiperspirant composition comprising:-
a) from 5 to 30 wt.%, preferably from 10 to 20 wt.% of an antiperspirant active;
b) from 1 to 5 wt.%, preferably from 1.25 to 4 wt.%, more preferably from 1.5 to 3 wt.% of a glyceryl mono- or di- ester;
c) from 0.5 to 5 wt.%, preferably from 1 to 4 wt.%, more preferably from 1.5 to 3 wt.% of a fatty alcohol;
d) from 1 to 8 wt.%, preferably from 2 to 6 wt.%, glycerol; and,
e) from 30 to 90 wt.% of water, preferably from 50 to 85 wt.%, more preferably from 60 to 80 wt.% of water,
(f) 0.15 to 2.5 wt %, preferably from 0.2 to 1 wt % of an ethoxylated fatty alcohol containing C14-C20 carbon atoms, wherein the degree of ethoxylation is between 10 to 25 moles of ethoxylate per mole of fatty alcohol;
wherein the weight ratio of b) to d) is from 1:3 to 3:1; and,
wherein the formulation has a viscosity as measured by a Brookfield viscometer at 25°C of from 1,000 to 8,000 mPa.s (cps).

### DETAILED DESCRIPTION OF THE INVENTION

The antiperspirant composition is aqueous. Water is present at a level of from 30 to 90 wt.%, preferably from 50 to 85 wt.%, more preferably from 60 to 80 wt.%. The composition may take on the form of an oil-in water emulsion.

The antiperspirant composition comprises an antiperspirant active.

Preferably the level of antiperspirant active present in the composition is from 5 to 30 wt.%, more preferably from 10 to 20 wt.%.

Preferably, the antiperspirant active comprises aluminium. Examples of aluminium containing antiperspirant active materials include: aluminium chlorohydrate, aluminium chloride, aluminium chlorohydrex polyethylene glycol, aluminium chlorohydrex propylene glycol, aluminium dichlorohydrate, aluminium dichlorohydrex polyethylene glycol, aluminium dichlorohydrex propylene glycol, aluminium sesquichlorohydrate, aluminium sesquichlorohydrate polyethylene glycol, aluminium sesquichlorohydrate propylene glycol, aluminium-zirconium octachlorohydrate, aluminium-zirconium octachlorohydrex gly, aluminium- zirconium pentachlorohydrate, aluminium-zirconium pentachlorohydrex gly, aluminium- zirconium tetrachlorohydrate, aluminium-zirconium tetrachlorohydrex gly, aluminium-zirconium trichlorohydrate, aluminium-zirconium trichlorohydrex gly, and combinations thereof.

Preferably the aluminium antiperspirant active is water soluble. More preferably the aluminium antiperspirant active is aluminium chlorohydrate (ACH).

The composition contains from 1 to 5 wt.%, preferably from 1.25 to 4 wt.%, more preferably from 1.5 to 3 wt.% of a glyceryl mono- or di- ester.

Glyceryl esters are esters of glycerol. Glycerol is a trihydric alcohol; the glyceryl ester is a mono- or, di- ester of glycerol. These esters can be the same or different. To form the glyceryl ester, the alcohol of the glycerol is reacted with a carboxylic acid species. Preferred glyceryl esters use glycerol reacted with fatty acids.

Preferably the glyceryl mono- or di- ester is a glyceryl ester of a C₁₂-C₂₂ fatty acid, preferably a glyceryl ester of a saturated C₁₂-C₂₂ fatty acid. More preferably, the glyceryl ester is a glyceryl monoester of a C₁₂-C₂₂ fatty acid, preferably glyceryl monostearate.

The composition comprises from 0.5 to 5 wt.%, preferably from 1 to 4 wt.%, more preferably from 1.5 to 3 wt.% of a fatty alcohol.

Fatty alcohols generally comprise long chain alkyl groups terminating in a primary alcohol. The long chain alkyl group can be for example C₈ to C₂₄. The alkyl group may be straight chain or branched, and may be saturated or unsaturated.

Preferably the fatty alcohol is a straight chain alkyl which is unsaturated. In the invention as claimed, the fatty alcohol comprises a C₁₀-C₂₀ fatty alcohol, preferably C₁₂-C₁₈ fatty alcohol.

The composition comprises from 1 to 8 wt.%, preferably from 2 to 6 wt.% of glycerol. Glycerol is a trihydric alcohol and can act as a humectant (another name for a moisturiser in the personal care field).

The composition may optionally comprise an emollient or one or more further humectants (other than glycerol) to aid the sensory profile. If present, then this optional material is preferably included at a level of from 0.5 to 20 wt.%, preferably from 1 to 10 wt.%. These values would include any glycerol already included in the formulation.

The composition comprises from 0.15 to 2.5 wt.%, more preferably from 0.2 to 1 wt.% of an ethoxylated fatty alcohol containing C₁₄-C₂₀ carbon atoms, wherein the degree of ethoxylation is between 10 to 25 moles of ethoxylate per mole of fatty alcohol. A preferred material is steareth-20, which is stearic acid a (C₁₈ fatty acid) ethoxylated with an average of 20 moles of ethoxylation.

The composition preferably comprises from 1 to 8 wt.%, preferably from 1.5 to 6 wt.%, more preferably from 2 to 5 wt.% of a triglyceride oil.

The triglyceride oil is a glycerol having three ester linkages.

Preferably the triglyceride oil comprises glycerol that have been reacted with three C₁₄-C₂₀ fatty acids. More preferably the triglyceride oil comprises glycerol that have been reacted with three with C₁₆-C₁₈ saturated and/or unsaturated fatty acids. Even more preferably the triglyceride oil comprises unsaturated C₁₆-C₁₈ fatty acids.

The triglyceride oil is most preferably sunflower seed oil.

The weight ratio of (b), the glyceryl mono- or di- ester, to (d), glycerol, is from 1:3 to 3:1, preferably from 1:2.5 to 2.5:1, more preferably from 2:1 to 1:2.

Preferably, the weight ratio of (b), the glyceryl mono- or di- ester, to (c), the fatty alcohol, is from 1:2 to 2:1, preferably from 1.5:1 to 1:1.5, more preferably from 1.2:1 to 1:1.2.

Preferably, the weight ratio of (c), the fatty alcohol, to (d) glycerol is from 1:3 to 3:1, preferably from 1:2.5 to 2.5:1, more preferably from 2:1 to 1:2.

If a triglyceride oil is included, then preferably, the weight ratio of (c), the fatty alcohol, to the triglyceride oil is from 1:3 to 3:1, preferably from 1:2.5 to 2.5:1, more preferably from 2:1 to 1:2.

Typically the antiperspirant composition may comprise a perfume. This may be in the form of a free perfume oil or mixture of perfume oils, or in the form of an encapsulated perfume.

Where perfume and triglyceride oil is included, then preferably the weight ratio of triglyceride oil to perfume is > 1:1, more preferably from 10:1 to 1.5:1, most preferably from 8:1 to 1.25:1.

Preferably, the formulation contains, on a wt.% basis, more of (b), the glyceryl mono- or di- ester, than, if present, any ethoxylated fatty alcohol containing C₁₄-C₂₀ carbon atoms, wherein the degree of ethoxylation is between 1 to 2 moles of ethoxylate per mole of fatty alcohol.

Said ethoxylated fatty alcohol if present, is preferably present at a level of less than 1 wt.%. Thus, the formulation preferably limits the amount of ethoxylated fatty alcohol having a low amount of ethoxylation to at most 1 wt.%. This has been found to provide a product that feels milder to the consumer, and displays a higher thermal stability as measured by differential scanning calorimetry (DSC).

The formulation preferably contains less than 1 wt.%, preferably less than 0.5 wt.%, more preferably less than 0.1 wt.%, even more preferably less than 0.05 wt.%, more preferably is free from an ethoxylated fatty alcohol containing C₁₄-C₂₀ carbon atoms, wherein the degree of ethoxylation is between 1 to 2 moles of ethoxylate per mole of fatty alcohol.

In some cases the degree of ethoxylation may be between 1 to 4 moles of ethoxylate per mole of fatty alcohol.

The composition may optionally comprise additional ingredients such as stabilisers, preservatives, antioxidants and the like. Typical inclusion levels of each may be from 0.01 to 1 wt.%. Examples of such materials are salts of ethylene diamine tetraacetic acid (EDTA) and pentaerythrityl tetra-di-t-butyl hydroxyhydrocinnamate.

The composition is an aqueous antiperspirant formulation. This composition may be dispensed to the consumer in many forms. A preferred dispenser to dispense the formulation to the consumer is a roll-on deodorant product comprising: a roll-on deodorant package comprising a roll-ball for dispensing an aqueous composition; and, the antiperspirant composition as described herein according to the invention.

The composition has a viscosity of between 1,000 and 8,000 mPa.s (cps), preferably between 3,000 and 8,000, more preferably between 3,500 to 8,000. These values are as measured on a Brookfield viscometer (Model DV-I+, RV3 spindle at 10rpm for 30 seconds at 25°C).

The formulations can be manufactured using the following process. The oils, including glyceryl mono- or di- ester, fatty alcohol, glycerol and any further optional oils such as ethoxylated fatty alcohol or triglyceride oil, are heated at 60°C to produce a melt. This is slowly added over a few minutes under shear to water heated to 60°C. The mixture is maintained with shear at 60°C for a few minutes, and subsequently cooled to about 58°C under shear for about 10 minutes. After cooling to about 50°C, the aluminium salt as a solution in water (in this case ACH) is added over a few minutes under shear. The mixture is cooled to about 40°C and any minors such as perfume, antioxidants are then added. The formulation can then be discharged from the mixing vessel into the packaging.

The formulation exhibits improved viscosity over current products. The lower viscosity allows the formulations to be used in conjunction with a roll-on dispenser.

The formulation also exhibits improved thermal stability over current products. The formulation of the invention is stable at 50°C or even 55°C without phase separation for at least 7 days. The formulation of the invention has been found to have a higher melting peak, measured by DSC to be 59°C.

### Examples

### Example 1 - comparison of thermal stability against current in-market product

The formulation of the invention (1) has been compared to a current in-market product (A) as a comparative example to check the thermal stability. The formulations were tested at 50°C. The formulations are shown in table 1.

**Table 1**

| | **Invention Formulation 1 (wt.%)** | **In-market Formulation A (wt.%)** |
|---|---|---|
| Aqua | 56.525 | 57.799 |
| Aluminium chlorohydrate 50% solution in aqua | 30.000 | 30.000 |
| Sunflower seed oil | 4.000 | 4.000 |
| Glycerol | 4.000 | 4.000 |
| Steareth-20 | 0.325 | 0.600 |
| Disodium EDTA | 0.100 | - |
| Pentaerythrityl tetra-di-t-butyl hydroxyhydrocinnamate | 0.050 | - |
| Glyceryl monostearate | 2.000 | - |
| Cetearyl alcohol | 2.000 | - |
| Steareth-2 | - | 2.600 |
| Vitamine E acetate | - | 0.001 |
| Perfume | 1.000 | 1.000 |

### Stability test at 50°C

Formulations 1 and A were tested for thermal stability by keeping them in a temperature controlled environment for a specified length of time measured in days.

Product A (comparative) started to display instability from day 4, and phase separated on day 6.

Product 1 (invention) was completely stable even after 84 days at 50°C.

### Example 2 - comparison of thermal stability

The formulation of the invention (1) has been compared to an antiperspirant lotion (B) which is comparative. Although this lotion contains the specified combination of materials, namely a glyceryl mono- or di- ester, a fatty alcohol and a triglyceride oil, they are not present in the required levels and/or weight ratios. The formulations are shown in table 2.

**Table 2**

| | **Invention Formulation 1 (wt.%)** | **In-market Lotion Formulation B (wt.%)** |
|---|---|---|
| Aqua | 56.525 | 51.600 |
| Aluminium chlorohydrate 50% solution in aqua | 30.000 | 30.000 |
| Sunflower seed oil | 4.000 | 1.000 |
| Glycerol | 4.000 | 2.000 |
| Steareth-20 | 0.325 | 1.310 |
| Disodium EDTA | 0.100 | - |
| Pentaerythrityl tetra-di-t-butyl hydroxyhydrocinnamate | 0.050 | - |
| Glyceryl monostearate | 2.000 | 7.500 |
| Cetearyl alcohol | 2.000 | 1.000 |
| Perfume | 1.000 | 1.200 |
| TiO₂ | - | 0.200 |
| Stearyl Alcohol | - | 3.500 |
| Steareth-2 | - | 0.190 |
| Silicone crosspolymer | - | 0.500 |
| Weight ratio of (b) to (c) | 1:1 | 1.67:1 |
| Weight ratio of (b) to (d) | 1:2 | 3.75:1 |
| Weight ratio of (c) to (d) | 1:2 | 2.25:1 |

### Thermal Stability measured by DSC

The phase transition temperature was measured by differential scanning calorimetry (DSC).

Measurements were performed in a Discovery Series DSC (TA Instruments) and analysed using Trios software (v3.3.1.4246). Samples were measured in aluminium pans and lids with a capacity of -180°C - 600°C (pan vol 40ul, 2-3 atmosp. Internal pressure). A measurement range from 10°C to 90°C and a heating rate of 10°C/min was used. Formulation B showed a much lower onset temperature of phase transition of <45°C. Formulation 1 had a much higher onset temperature of phase transition of 57°C, with a peak value of 59°C.

### Example 3 - Measure of Viscosity

Viscosity measurements for formulations 1 and comparative formulation A were carried out on a Brookfield viscometer (Model DV-I+, RV3 spindle at 10 rpm for 30 seconds) at 25°C. The results are shown in table 3.

**Table 3**

| | Brookfield Viscosity at 10rpm |
|---|---|
| Formulation 1 | 3,400 cps |
| Formulation A (comparative) | 38,600 cps |

As can be clearly seen from Table 3, the comparative example, which had a much higher ratio of b) to d) than the formulation according to the invention also had a much higher viscosity.

The viscosity value for the formulation A (comparative example) is too high (i.e. too viscous) to be efficiently dispensed from a roll-on antiperspirant product.

## Claims

1. A roll-on deodorant product comprising: a roll-on deodorant package comprising a roll-ball for dispensing an aqueous antiperspirant composition; the composition comprising:-
a) from 5 to 30 weight percent, preferably from 10 to 20 weight percent of an antiperspirant active;
b) from 1 to 5 weight percent, preferably from 1.25 to 4 weight percent, more preferably from 1.5 to 3 weight percent of a glyceryl mono- or di- ester;
c) from 0.5 to 5 weight percent, preferably from 1 to 4 weight percent, more preferably from 1.5 to 3 weight percent of a fatty alcohol, comprising a C₁₀-C₂₀ fatty alcohol;
d) from 1 to 8 weight percent, preferably from 2 to 6 weight percent, of glycerol; and,
e) from 30 to 90 weight percent, preferably from 50 to 85 weight percent, more preferably from 60 to 80 weight percent of water;
f) 0.15 to 2.5 weight percent, preferably from 0.2 to 1 weight percent of an ethoxylated fatty alcohol containing C₁₄-C₂₀ carbon atoms, wherein the degree of ethoxylation is between 10 to 25 moles of ethoxylate per mole of fatty alcohol;
wherein the weight ratio of b) to d) is from 1:3 to 3:1; and,
wherein the formulation has a viscosity as measured by a Brookfield viscometer at 25 degrees centigrade of from 1,000 to 8,000 mPa.s (cps) as measured with the method of the description.

2. A composition according to claim 1, wherein the antiperspirant active is an aluminium salt, preferably a water soluble aluminium salt, most preferably aluminium chlorohydrate (ACH).

3. A composition according to claim 1 or claim 2, wherein the weight ratio of b) to c) is from 2:1 to 1:2, preferably from 1.5:1 to 1:1.5, more preferably from 1.2:1 to 1:1.2.

4. A composition according to any preceding claim, wherein the weight ratio of b) to d) is from 1:2.5 to 2.5:1, more preferably from 2:1 to 1:2.

5. A composition according to any preceding claim, wherein the glyceryl mono- or di-ester is a glyceryl ester of a C12-C22 fatty acid, preferably a glyceryl ester of a saturated C12-C22 fatty acid.

6. A composition according to any preceding claim, wherein the glyceryl ester is a glyceryl monoester of a C₁₂-C₂₂ fatty acid, preferably glyceryl monostearate.

7. A composition according to any preceding claim, wherein the fatty alcohol is a C₁₂-C₁₈ fatty alcohol.

8. A composition according to any preceding claim, wherein the viscosity is from 3,000 to 8,000 mPa.s (cps), preferably from 3,500 to 8,000 mPa.s (cps).

9. A composition according to any preceding claim, additionally comprising from 1 to 8 weight percent, preferably 1.5 to 6 weight percent, more preferably from 2 to 5 weight percent of a triglyceride oil.

10. A composition according to claim 9, wherein the triglyceride oil comprises C₁₄-C₂₀ fatty acids, preferably C₁₆-C₁₈ saturated and/or unsaturated fatty acids, more preferably the triglyceride oil is sunflower seed oil.

11. A composition according to any preceding claim, wherein the weight ratio of c) to d) is from 1:3 to 2:1, preferably from 1:2.5 to 1.5:1, more preferably from 1:2.5 to 1:1.

12. A composition according to any preceding claim, which comprises less than 1 weight percent, preferably 0.5 weight percent, more preferably 0.1 weight percent, even more preferably less than 0.05 weight percent, most preferably is free from an ethoxylated fatty alcohol containing C₁₄-C₂₀ carbon atoms, wherein the degree of ethoxylation is between 1 to 2 moles of ethoxylate per mole of fatty alcohol.

13. A composition according to any preceding claim, additionally comprising perfume, wherein preferably the weight ratio of triglyceride oil to perfume is > 1:1, preferably from 10:1 to 1.5:1, more preferably from 8:1 to 1.25:1.

## Patentansprüche

1. Roll-on-Deodorantprodukt, umfassend: eine Roll-on-Deodorantpackung, umfassend einen Roll-Ball zum Abgeben einer wässrigen Antiperspirant-Zusammensetzung, wobei die Zusammensetzung umfasst:
a) 5 bis 30 Gewichtsprozent, bevorzugt 10 bis 20 Gewichtsprozent, eines Antiperspirant-Wirkstoffs;
b) 1 bis 5 Gewichtsprozent, bevorzugt 1,25 bis 4 Gewichtsprozent, bevorzugter 1,5 bis 3 Gewichtsprozent, eines Glycerylmono- oder -diesters;
c) 0,5 bis 5 Gewichtsprozent, bevorzugt 1 bis 4 Gewichtsprozent, bevorzugter 1,5 bis 3 Gewichtsprozent, eines Fettalkohols, umfassend einen C₁₀-C₂₀-Fettalkohol;
d) 1 bis 8 Gewichtsprozent, bevorzugt 2 bis 6 Gewichtsprozent, Glycerin; und
e) 30 bis 90 Gewichtsprozent, bevorzugt 50 bis 85 Gewichtsprozent, bevorzugter 60 bis 80 Gewichtsprozent, Wasser;
f) 0,15 bis 2,5 Gewichtsprozent, bevorzugt 0,2 bis 1 Gewichtsprozent, eines ethoxylierten Fettalkohols enthaltend C₁₄-C₂₀ Kohlenstoffatome, wobei der Ethoxylierungsgrad zwischen 10 bis 25 mol Ethoxylat pro mol Fettalkohol liegt;
wobei das Gewichtsverhältnis von b) zu d) 1:3 bis 3:1 beträgt; und
wobei die Formulierung eine Viskosität, gemessen mit einem Brookfield-Viskosimeter bei 25 Grad Celsius von 1.000 bis 8.000 mPa.s (cps), gemessen mit dem Verfahren der Beschreibung, aufweist.

2. Zusammensetzung nach Anspruch 1, wobei der Antiperspirant-Wirkstoff ein Aluminiumsalz, bevorzugt ein wasserlösliches Aluminiumsalz, am meisten bevorzugt Aluminiumchlorhydrat (ACH), ist.

3. Zusammensetzung nach Anspruch 1 oder Anspruch 2, wobei das Gewichtsverhältnis von b) zu c) 2:1 bis 1:2, bevorzugt 1,5:1 bis 1:1,5, bevorzugter 1,2:1 bis 1:1,2, beträgt.

4. Zusammensetzung nach irgendeinem vorhergehenden Anspruch, wobei das Gewichtsverhältnis von b) zu d) 1:2,5 bis 2,5:1, bevorzugter 2:1 bis 1:2, beträgt.

5. Zusammensetzung nach irgendeinem vorhergehenden Anspruch, wobei der Glycerylmono- oder -diester ein Glycerylester einer C₁₂-C₂₂-Fettsäure, bevorzugt ein Glycerylester einer gesättigten C₁₂-C₂₂-Fettsäure, ist.

6. Zusammensetzung nach irgendeinem vorhergehenden Anspruch, wobei der Glycerylester ein Glycerylmonoester einer C₁₂-C₂₂-Fettsäure, bevorzugt Glycerylmonostearat, ist.

7. Zusammensetzung nach irgendeinem vorhergehenden Anspruch, wobei der Fettalkohol ein C₁₂-C₁₈-Fettalkohol ist.

8. Zusammensetzung nach irgendeinem vorhergehenden Anspruch, wobei die Viskosität 3.000 bis 8.000 mPa.s (cps), bevorzugt 3.500 bis 8.000 mPa.s (cps), beträgt.

9. Zusammensetzung nach irgendeinem vorhergehenden Anspruch, zusätzlich umfassend 1 bis 8 Gewichtsprozent, bevorzugt 1,5 bis 6 Gewichtsprozent, bevorzugter 2 bis 5 Gewichtsprozent, eines Triglyceridöls.

10. Zusammensetzung nach Anspruch 9, wobei das Triglyceridöl C₁₄-C₂₀-Fettsäuren, bevorzugt gesättigte und/oder ungesättigte C₁₆-C₁₈-Fettsäuren, umfasst, wobei das Triglyceridöl bevorzugter Sonnenblumenkernöl ist.

11. Zusammensetzung nach irgendeinem vorhergehenden Anspruch, wobei das Gewichtsverhältnis von c) zu d) 1:3 bis 2:1, bevorzugt 1:2,5 bis 1,5:1, bevorzugter 1:2,5 bis 1:1, beträgt.

12. Zusammensetzung nach irgendeinem vorhergehenden Anspruch, die weniger als 1 Gewichtsprozent, bevorzugt 0,5 Gewichtsprozent, bevorzugter 0,1 Gewichtsprozent, noch bevorzugter weniger als 0,05 Gewichtsprozent, von einem ethoxylierten Fettalkohol, der C₁₄-C₂₀ Kohlenstoffatome enthält, wobei der Ethoxylierungsgrad zwischen 1 bis 2 mol Ethoxylat pro mol Fettalkohol liegt, umfasst und am meisten bevorzugt frei davon ist.

13. Zusammensetzung nach irgendeinem vorhergehenden Anspruch, zusätzlich umfassend Duftstoff, wobei bevorzugt das Gewichtsverhältnis von Triglyceridöl zu Duftstoff > 1:1, bevorzugt 10:1 bis 1,5:1, bevorzugter 8:1 bis 1,25:1, ist.

## Revendications

1. Produit de déodorant à bille comprenant : un emballage de déodorant à bille comprenant une bille pour distribuer une composition d'antiperspirant aqueuse ; la composition comprenant :
a) de 5 à 30 pourcents en masse, de préférence de 10 à 20 pourcents en masse d'un actif d'antiperspirant ;
b) de 1 à 5 pourcents en masse, de préférence de 1,25 à 4 pourcents en masse, encore mieux de 1,5 à 3 pourcents en masse d'un mono- ou di-ester glycérylique ;
c) de 0,5 à 5 pourcents en masse, de préférence de 1 à 4 pourcents en masse, encore mieux de 1,5 à 3 pourcents en masse d'un alcool gras, comprenant un alcool gras en C₁₀-C₂₀ ;
d) de 1 à 8 pourcents en masse, de préférence de 2 à 6 pourcents en masse de glycérol ; et,
e) de 30 à 90 pourcents en masse, de préférence de 50 à 85 pourcents en masse, encore mieux de 60 à 80 pourcents en masse d'eau ;
f) 0,15 à 2,5 pourcents en masse, de préférence de 0,2 à 1 pourcent en masse d'un alcool gras éthoxylé contenant C₁₄-C₂₀ atomes de carbone, où le degré d'éthoxylation est de 10 à 25 moles d'éthoxylate par mole d'alcool gras ;
où le rapport en masse de b) à d) est de 1:3 à 3:1 ; et,
où la formulation a une viscosité comme mesurée par un viscosimètre Brookfield à 25 degrés Centigrade de 1 000 à 8 000 mPa.s (cps) comme mesurée avec la méthode de la description.

2. Composition selon la revendication 1, où l'actif d'antisperspirant est un sel d'aluminium, de préférence un sel d'aluminium soluble dans l'eau, encore mieux du chlorohydrate d'aluminium (ACH).

3. Composition selon la revendication 1 ou revendication 2, où le rapport massique de b) à c) est de 2:1 à 1:2, de préférence de 1,5:1 à 1:1,5, encore mieux de 1,2:1 à 1:1,2.

4. Composition selon l'une quelconque des revendications précédentes, où le rapport massique de b) à d) est de 1:2,5 à 2,5:1, encore mieux de 2:1 à 1:2.

5. Composition selon l'une quelconque des revendications précédentes, où le mono- ou di-ester glycérylique est un ester glycérylique d'un acide gras en C₁₂-C₂₂, de préférence un ester glycérylique d'un acide gras en C₁₂-C₂₂ saturé.

6. Composition selon l'une quelconque des revendications précédentes, où l'ester glycérylique est un monoester glycérylique d'un acide gras en C₁₂-C₂₂, de préférence le monostéarate de glycéryle.

7. Composition selon l'une quelconque des revendications précédentes, où l'alcool gras est un alcool gras en C₁₂-C₁₈.

8. Composition selon l'une quelconque des revendications précédentes, où la viscosité est de 3 000 à 8 000 mPa.s (cps), de préférence de 3 500 à 8 000 mPa.s (cps).

9. Composition selon l'une quelconque des revendications précédentes, comprenant de plus de 1 à 8 pourcents en masse, de préférence de 1,5 à 6 pourcents en masse, encore mieux de 2 à 5 pourcents en masse d'une huile de triglycéride.

10. Composition selon la revendication 9, où l'huile de triglycéride comprend des acides gras en C₁₄-C₂₀, de préférence des acides gras saturés et/ou insaturés en C₁₆-C₁₈, encore mieux l'huile de triglycéride est une huile de graines de tournesol.

11. Composition selon l'une quelconque des revendications précédentes, où le rapport massique de c) à d) est de 1:3 à 2:1, de préférence de 1:2,5 à 1,5:1, encore mieux de 1:2,5 à 1:1.

12. Composition selon l'une quelconque des revendications précédentes, qui comprend moins de 1 pourcent en masse, de préférence 0,5 pourcent en masse, encore mieux 0,1 pourcent en masse, bien mieux encore moins de 0,05 pourcent en masse, plus particulièrement est exempte d'un alcool gras éthoxylé contenant C₁₄-C₂₀ atomes de carbone, où le degré d'éthoxylation est de 1 à 2 moles d'éthoxylate par mole d'alcool gras.

13. Composition selon l'une quelconque des revendications précédentes, comprenant de plus du parfum, où de préférence le rapport massique d'huile de triglycéride à parfum est > 1:1, de préférence de 10:1 à 1,5:1, encore mieux de 8:1 à 1,25:1.
